# EUROPÄISCHE PATENTSCHRIFT

(11) **EP 0 681 814 B1**
(45) Veröffentlichungstag und Bekanntmachung des Hinweises auf die Patenterteilung: **31.05.2000**
(21) Anmeldenummer: 95103890.0
(22) Anmeldetag: 16.03.1995
(51) Int. Cl.: A61F 2/12, C08L 83/00, A61L 27/00, A61F 2/52

(54) **Verfahren zur Herstellung von Brustprothesen**
Process for the production of breast prosthesis
Procédé pour la fabrication d'une prothèse de sein

(30) Priorität: 15.04.1994 DE 4413076
(43) Veröffentlichungstag der Anmeldung: 15.11.1995
(73) Patentinhaber: AMOENA Medizin-Orthopädie-Technik GmbH, D-83064 Raubling (DE)
(72) Erfinder: Wild, Helmut, D-83115 Neubeuern (DE)
(74) Vertreter: Lorenz, Eduard

(56) Entgegenhaltungen:
- EP-A- 0 054 197
- DE-U- 9 201 918
- US-A- 4 380 569
- US-A- 5 340 352
- DATABASE WPI Week 9326 Derwent Publications Ltd., London, GB; AN 93-208311 & US-A-5340352 & JP-A-05 131 007

## Beschreibung

Die Erfindung betrifft ein Verfahren zur Herstellung von Brustprothesen aus einem schalenförmigen Körper aus einer additionsvernetzenden Zwei-Komponenten-Silikon-Kautschuk-Masse, der in eine diesen einen einhüllende Kunststoffolie eingefaßt ist, bei dem der Zwei-Komponenten-Silikon-Kautschuk-Masse ein aus Hohlkugeln oder Mikrokugeln bestehender Füllstoff mit geringerer Dichte beigemengt wird und die Mischung in eine Hülle aus Kunststoff-Folie eingefüllt wird.

Brustprothesen dieser Art sind aus dem DE-GM 92 01 918 bekannt. Diese Brustprothesen werden nach dem üblichen Verfahren hergestellt, nach dem die beiden Komponenten des Silikon-Kautschuks in ihrer Viskosität so eingestellt sind, daß sie zur Füllung der bereits zu den Prothesenhüllen miteinander verschweißten Kunststoffolien zusammen mit dem Füllstoff gepumpt und durch einen Mischer geleitet werden können. Um pumpfähig zu sein, müssen die beiden Komponenten bzw. die Mischung eine verhältnismäßig geringe Viskosität aufweisen, die dazu führt, daß die leichteren Füllstoffkugeln in den in die Formen eingelegten beutelförmigen Kunststoffhüllen vor ihrem Aushärten aufschwimmen, so daß die gleichmäßige Verteilung des Füllstoffes in der Silikon-Kautschuk-Masse verloren geht und der Füllstoff aus den unteren Bereichen der Mischung nach oben wandert und sich dem oberen Bereich der beutelförmigen Kunststoffhüllen sammelt.

Aufgabe der Erfindung ist es, ein Verfahren der eingangs angegebenen Art vorzuschlagen, nach dem sich Leichtprothesen mit im wesentlichen gleichmäßiger Verteilung des Füllstoffes im Prothesenkörper herstellen lassen.

Diese Aufgabe wird durch die Merkmale des Anspruchs 1 gelöst. So können die beiden Komponenten der Silikon-Kautschuk-Masse mit dem Füllstoff gemischt und in ihre Viskosität so eingestellt werden, daß die Mischung eine Viskosität von 1.800 bis 2.200 mPa s und vorzugsweise etwas 2.000 mPa^{·}s ist sie so zäh, daß die Füllstoffkugeln bis zu ihrer Aushärtung in merklicher Weise nicht aufschwimmen können, so daß die gewünschte gleichmäßige Verteilung der Füllstoffe der Mischung und später in der ausgehärteten Prothese erhalten bleibt.

Die üblicherweise zur Herstellung der Brustprothesen verwendeten Komponenten des Silikon-Kautschuks bestehen aus einer Mischung eines Polymers, Silikon-Öl, einem Inhibitor, einem Vernetzer und einer Farbpaste unter Verwendung eines Katalysators. Dabei haben die Komponenten bei ihrer Verarbeitung eine Viskosität von etwa 500 - 600 mPa^{·}s bei einer Topfzeit von 4 bis 5 Stunden bei Raumtemperatur. Das Polymer besteht aus einem venylendgestoppten linearen Polydimethylsiloxan (Dimeticon). Als Silikon-Öl wird Polydimethylsiloxan ohne funktionelle Gruppen verwendet. Das Silikon-Öl dient der Verdünnung der Komponenten. Als Katalysator wird ein Platinkatalysator verwendet. Die Vernetzer sind Polysiloxane mit siliciumgebundenen Wasserstoffatomen. Lediglich zur Verbesserung des Aussehens kann eine Farbpigmentmischung verwendet werden, die zweckmäßigerweise dem Silikon-Öl zugesetzt wird.

Zur Herstellung der Leichtprothesen wird der Füllstoff einer oder vorzugsweise beiden Komponenten der Silikon-Kautschuk-Masse zugesetzt und in die Komponenten eingerührt und durch einen Statik- oder Dynamik-Mischer vermischt und anschließend in die aus zwei verschweißten Folien bestehende vorbereitete Kunststoffhülle eingefüllt. Die derart gefüllte Kunststoffhülle wird dann in der üblichen Weise in eine Form eingelegt und in dieser ausgehärtet. Die Einfüllöffnung in dem Hüllenrand kann nach dem Befüllen oder während des Aushärtens verschweißt werden.

Der Füllstoff kann aus Hohlkugeln aber auch aus Leichtstoffkugeln aus einem anderen geeigneten Material bestehen.

Bei einer Variante des erfindungsgemäßen Verfahren werden die vorstehend angegebenen üblichen Komponenten des Silikon-Kautschuks verwendet, die beiden Komponenten werden jedoch so eingestellt, daß die Silikon-Kautschuk-Füllstoffmischung eine Viskosität von etwa 2.000 mPa^{·}s besitzt.

## Patentansprüche

1. Verfahren zur Herstellung von Brustprothesen aus einem schalenförmigen Körper aus einer additionsvernetzenden Zwei-Komponenten-Silikon-Kautschuk-Masse, der in eine diesen einhüllende Kunststoffolie eingefaßt ist, bei dem der Zwei-Komponenten-Silikon-Kautschuk-Masse ein aus Hohlkugeln oder Mikrokugeln bestehender Füllstoff mit geringerer Dichte beigemengt wird und die Mischung in eine Hülle aus Kunststoff-Folie eingefüllt wird,
**dadurch gekennzeichnet**,
daß die beiden Komponenten der Silikon-Kautschuk-Masse mit den Hohlkugeln oder den Mikrokugeln gemischt und in ihrer Viskosität so eingestellt werden, daß die Mischung eine Viskosität von 1800 bis 2.200 mPa^{·}s hat, so daß die Füllstoffkugeln bis zur Aushärtung der Mischung nicht in merklicher Weise aufschäumen können und daß die Mischung in einer Form unter Wärmeeinwirkung ausgehärtet wird.

## Claims

1. Process for the production of breast prostheses from a shell-shaped body of an addition-crosslinked two-component silicone rubber compound which is enclosed in a plastics film enveloping it, in which process the two-component silicone rubber compound has admixed with it a filler of lower density, comprising hollow beads or microbeads, and the mixture is filled into an enclosure of plastics film, characterized in that the two components of the silicone rubber compound are mixed with the hollow beads or the microbeads and are adjusted in their viscosity such that the mixture has a viscosity of from 1800 to 2200 mPas, with the result that the filler beads cannot expand to any notable extent until the mixture cures, and in that the mixture is cured in a mould under the effect of heat.

## Revendications

1. Procédé pour la fabrication de prothèses du sein en un corps en forme de coupe à partir d'une masse de caoutchouc de silicone à deux composants réticulable par addition, qui est insérée dans une feuille de matière plastique l'enveloppant, où la masse de caoutchouc de silicone à deux composants est mélangée à une charge se composant de billes creuses ou de microbilles d'une densité minime et le mélange est introduit dans une enveloppe en une feuille en matière plastique,
**caractérisé en ce que,**
les deux composants de la masse de caoutchouc de silicone sont mélangés aux billes creuses ou aux microbilles et sont ajustés en viscosité de façon que le mélange ait une viscosité de 1800 à 2200 mPa.s pour que les billes de la charge, jusqu'au durcissement du mélange, ne puissent mousser d'une manière remarquable et ce que le mélange est durci dans un moule sous l'effet de la chaleur.
